# EUROPEAN PATENT SPECIFICATION

(11) **EP 2 957 321 B1**
(45) Date of publication and mention of the grant of the patent: **10.10.2018**
(21) Application number: 15170037.4
(22) Date of filing: 01.06.2015
(51) Int. Cl.: A61N 1/365, A61N 1/368, A61N 1/39

(54) **APPARATUS TO OPTIMIZE PACING PARAMETERS**
VORRICHTUNG ZUR OPTIMIERUNG VON SCHRITTMACHERPARAMETERN
APPAREIL PERMETTANT D'OPTIMISER DES PARAMÈTRES DE STIMULATION

(30) Priority: 19.06.2014 US 201462014134 P
(43) Date of publication of application: 23.12.2015
(73) Proprietor: BIOTRONIK SE & Co. KG, 12359 Berlin (DE)
(72) Inventor: Whittington, R. Hollis, Portland, OR 97202 (US); Müssig, Dirk, West Linn, 97068 (US)
(74) Representative: Galander, Marcus

(56) References cited:
- US-A1- 2005 182 447
- US-A1- 2010 004 712
- US-A1- 2012 203 090
- US-A1- 2013 053 912
- US-B1- 8 600 497

## Description

The invention refers to a heart stimulator comprising a stimulation control unit, one or more stimulation units, an impedance measurement unit and an impedance evaluation unit wherein the stimulation control unit is operatively connected to one or more stimulation units to control timing of stimulation pulses by said one or more stimulation units.

The invention particularly refers to a heart stimulator for delivering a cardiac resynchronization therapy (CRT). Such heart stimulator can be an implantable cardiac pacemaker or an implantable cardioverter defibrillator (CRT-D/ICD).

Cardiac resynchronization therapy (CRT) is emerging as one of the primary treatments for patients with heart failure (HF). Numerous methods have been proposed and/or developed for optimal programming of the CRT devices, with particular focus on two timing parameters: atrio-ventricular delay (AVD) and the inter-ventricular delay (VVD). Close attention is also paid to the site of stimulation, which is selectable during the lead implant procedure as well as via electrode selection using multi-electrode leads or catheters. Implantable heart stimulators can be used for cardiac rhythm management (CRM) for treating a variety of heart functional and rhythm disorders including but not limited to bradycardia, tachycardia or fibrillation by way of electric stimulation pulses delivered to the heart tissue, the myocardium. A sufficiently strong stimulation pulse outside a heart chamber's refractory period leads to excitation of the myocardium of that heart chamber, which in turn is followed by a contraction of the respective heart chamber.

Depending on the disorder to be treated, such heart stimulator generates electrical stimulation pulses that are delivered to the heart tissue (myocardium) of a respective heart chamber according to an adequate timing regime. Delivery of stimulation pulses to the myocardium is usually achieved by means of an electrode lead that is electrically connected to a stimulation pulse generator inside a heart stimulator's housing and that carries a stimulation electrode or multiple stimulation electrodes in the region of its distal end. A stimulation pulse also is called a pace. Similarly, pacing a heart chamber means stimulating a heart chamber by delivery of a stimulation pulse.

In order to be able to sense the contraction of a heart chamber which occurs naturally without artificial stimulation and which is called an intrinsic contraction, the heart stimulator usually includes at least one sensing stage that is connected to a sensing electrode and said electrode is placed in or near the heart chamber. An intrinsic excitation of a heart chamber results in characteristic electrical potentials that can be picked up via the sensing electrode and that can be evaluated by the sensing stage in order to determine whether an intrinsic excitation - called: intrinsic event - has occurred.

Usually, a heart stimulator features separate stimulation pulse generators for each heart chamber to be stimulated. Therefore, in a dual chamber pacemaker, usually an atrial and a ventricular stimulation pulse generator for generating atrial and ventricular stimulation pulses are provided. Delivery of an atrial or a ventricular stimulation pulse causing an artificial excitation of the atrium or the ventricle, respectively, is called an atrial stimulation event AP (atrial paced event) or a ventricular stimulation event VP (ventricular paced event), respectively.

Similarly, common heart stimulators feature separate sensing stages for each heart chamber to be of interest. In a dual chamber pacemaker usually two separate sensing stages, an atrial sensing stage and a ventricular sensing stage, are provided that are capable to detect intrinsic atrial events AS (atrial sensed event) or intrinsic ventricular events VS (ventricular sensed event), respectively.

In a heart cycle, an excitation of the myocardium leads to a depolarization of the myocardium that leads to a contraction of the heart chamber. If the myocardium is fully depolarized it is unsusceptible for further excitation and is thus refractory. Thereafter, the myocardium repolarizes and thus relaxes and the heart chamber expands again. In a typical intracardiac electrogram (iEGM) depolarization of the ventricle corresponds to a signal known as the "R-wave". The repolarization of the ventricular myocardium coincides with a signal known as the "T-wave". Atrial depolarization is manifested by a signal known as the "P-wave".

In a healthy heart, initiation of the cardiac cycle normally begins with depolarization of the sinoatrial (SA) node. This specialized structure is located in the upper portion of the right atrium wall and acts as a natural "pacemaker" of the heart. In a normal cardiac cycle and in response to the initiating SA depolarization, the right atrium contracts and forces the blood that has accumulated therein into the ventricle. The natural stimulus causing the right atrium to contract is conducted to right ventricle via the atrioventricular node (AV node) with a short, natural delay, the atrioventricular delay (AV-delay). Thus, a short time after the right atrial contraction (a time sufficient to allow the bulk of the blood in the right atrium to flow through the one-way valve into the right ventricle), the right ventricle contracts, forcing the blood out of the right ventricle to body tissue. A typical time interval between contraction of the right atrium and contraction of the right ventricle might be 100 ms; a typical time interval between contraction of the right ventricle and the next contraction of the right atrium might be 800 ms. Thus, it is an right atrial contraction (A), followed a relatively short time thereafter by a right ventricle contraction (V), followed a relatively long time thereafter by the next right atrial contraction, that produces the desired AV synchrony. Where AV synchrony exists, the heart functions very efficiently as a pump in delivering life-sustaining blood to body tissue; where AV synchrony is absent, the heart functions as an inefficient pump (largely because the right ventricle is contracting when it is not filled with blood).

Similarly, the left ventricle contracts in synchrony with right atrium and the right ventricle wit a positive or negative time delay between a right ventricular contraction and a left ventricular contraction.

A pacemaker generally shall induce a contraction of a heart chamber by delivery of a stimulation pulse (pacing pulse) to said chamber when no natural (intrinsic) contraction of said chamber occurs in due time. A contraction of a heart chamber often is called "event". Since a contraction may be an intrinsic contraction, which can be sensed by an according sensing stage of a pacemaker, such event is called a sensed event. A contraction due to delivery of a stimulation pulse is called a paced event. A sensed event in the atrium is called As, a paced atrial event is called Ap. Similarly, a sensed event in the ventricle is called Vs and a paced ventricular event is called Vp.

To mimic the natural behavior of a heart, a dual-chamber pacemaker provides for an AV-delay timer to provide for an adequate time delay (atrioventricular delay, AV-delay, AVD) between a natural (intrinsic) or a stimulated (paced) right atrial contraction and a right ventricular contraction. In a similar way a biventricular pacemaker provides for an adequate time delay (VV-delay, VVD) between a right ventricular contraction and a left ventricular contraction.

The time delay for a left ventricular (stimulated, paced) contraction may be timed from a scheduled right ventricular contraction which has not yet occurred or from a natural (intrinsic) or a stimulated (paced) right atrial contraction. In the latter case a left ventricular stimulation pulse is scheduled by a time interval AVD + VVD.

To deal with possibly occurring natural (intrinsic) atrial or ventricular contractions, a demand pacemaker schedules a stimulation pulse for delivery at the end of the AV-delay or the VV-delay, respectively. The delivery of said stimulation pulse is inhibited, is a natural contraction of the heart chamber to be stimulated is sensed within the respective time delay.

A natural contraction of a heart chamber can be similarly detected by the evaluating electrical signals sensed by the sensing channels. In the sensed electrical signal the depolarization of an atrium muscle tissue is manifested by occurrence of a P-wave. Similarly, the depolarization of ventricular muscle tissue is manifested by the occurrence of a R-wave. The detection of a P-wave or a R-wave signifies the occurrence of intrinsic atrial, As, or ventricular, Vs events, respectively.

A dual chamber pacemaker featuring an atrial and a ventricular sensing stage and an atrial and a ventricular stimulation pulse generator can be operated in a number of stimulation modes like VVI, wherein atrial sense events are ignored and no atrial stimulation pulses are generated, but only ventricular stimulation pulses are delivered in a demand mode, AAI, wherein ventricular sense events are ignored and no ventricular stimulation pulses are generated, but only atrial stimulation pulses are delivered in a demand mode, or DDD, wherein both, atrial and ventricular stimulation pulses are delivered in a demand mode. In such DDD mode of pacing, ventricular stimulation pulses can be generated in synchrony with sensed intrinsic atrial events and thus in synchrony with an intrinsic atrial rate, wherein a ventricular stimulation pulse is scheduled to follow an intrinsic atrial contraction after an appropriate atrioventricular delay (AV-delay; AVD), thereby maintaining the hemodynamic benefit of atrioventricular synchrony.

The AV-delay determines the chronological relation between an atrial event and a prescribed point of time of a ventricular event, the ventricular escape interval.

Since an optimal AV-delay may vary for different heart rates or stimulation rates and may even vary from patient to patient, the AV-delay usually is adjustable.

In order to promote natural ventricular events, often the ventricular escape interval is extended by a short time interval thus resulting in a prolonged ventricular escape interval called "AV hysteresis interval"

Ventricular pacing in one or both ventricles is required for patients with AV-block and for patients who exhibit congestive heart failure (CHF patients) that are indicated for cardiac resynchronization therapy (CRT).

Heart failure (HF) is a progressive disease that ultimately leads to ventricular dysfunction. Through the course of ventricular remodeling process, the ventricular geometry changes, which further cause conduction system abnormalities, for example, the inter- or intraventricular conduction delays. Consequently, contraction is no longer organized or synchronous between the left ventricle (LV) and right ventricle (RV), leading to ventricular dyssynchrony.

The CRT is a proven technique for treating HF patients with cardiac dyssynchrony. In addition to conventional rightventricular pacing, the left ventricle is also paced by implanting a pacing lead through a transvenous approach via the coronary sinus (CS). By electrically stimulating the site of late ventricular activation, forcing the right ventricle and the left ventricle to contract in a synchronized manner, the CRT can create a synchronized pumping of the ventricles, thus increasing the efficiency of ventricular contraction and improving the hemodynamics.

Nonetheless, clinical experience has shown that about one third of the CRT candidates are non-respondents, and the possible reasons may include inappropriate CS lead location, regional LV infarction, non-optimal pacing parameter settings, etc. Evidence has shown that optimizing pacing parameters, particularly the AVD and VVD, may change some CRT non-respondents to respondents. Even for the respondents, the AVD and VVD optimization may further improve the efficacy of the CRT.

CRT optimization can be done through echo technique such as tissue Doppler imaging. CRT optimization can also be achieved based on some metrics derived from the ECG or EGM signal, such as the P wave duration and/or QRS duration. Alternatively, the device settings can be optimized based on hemodynamic indexes that are assumed to correlate to the stroke volume or cardiac output, such as the blood pressure or its temporal derivative, the blood oxygen saturation, blood pH, etc. In addition, CRT optimization has been proposed by means of intracardiac impedance measurement which serves a surrogate of ventricular volume.

The AVD optimization, which aims to maintain synchronized timing between the atria and ventricles, has been a focus of research since the early development of the dual-chamber pacemakers two decades ago. The introduction of CRT in the late 1990s has generated further interests in AVD optimization. In addition, it has brought a new challenge for VVD optimization, which aims to coordinate RV and LV contractions. Moreover, the AVD and VVD optimizations have been further complicated by other confounding factors, for example the pacing rate and the pacing mode.

Numerous methods have been proposed and/or developed for optimal programming of the AVD and VVD in CRT devices.

For patients with intact sinus rhythm or with effective atrial pacing it is beneficial to stimulate the ventricle(s) synchronous with the atrial activation, i.e., after a certain delay period after the atrial event. Standard AV-synchronous dual- or three-chamber implantable devices have a programmable AVD that can be adjusted by the physician. Several studies have shown the importance of individual AVD optimization to improve the cardiac output. Especially for CHF patients an optimization of the AVD is essential. As the pumping efficacy is impaired, the optimal timing of the ventricular stimulus in relation to the atrial event contributes significantly to the cardiac performance. If the AVD is too short, the ventricle contracts before it is completely filled by the atrial blood inflow. The active filling time is reduced. Hence the stroke volume and the cardiac output is reduced. If the AVD is too long, the ventricle contracts a while after the closure of the atrioventricular valve. Hence the passive filling time of the ventricle, i.e., the diastolic filling period during the myocardial relaxation before the atrial kick, is decreased. Also backflow of blood from the ventricle into the atrium, e.g., mitral regurgitation, is likely. Thus also in this case the cardiac output is reduced. Similar to the heart rate also the optimal AVD depends on the activation state of the circulation. If the sympathetic tone is high, e.g., during exercise, the optimal AVD is shortened compared to the resting value.

Several methods for individual AVD optimization are state of the art. The adjustment of AVD in most cases is performed during the follow-up procedure by the physician with external measurement systems, not by the implant itself. In most of the methods the patient is in rest during the adjustment procedure and only the 'static' AVD is optimized. Although modern pacing devices possess a programmable dynamic AVD, i.e., an AVD that depends on the heart rate, the dynamic values are estimated in the majority of cases.

Conventionally, the AVD optimization in clinical practice has been achieved using echocardiographic techniques, particularly by measuring the pulse-wave Doppler signals of the mitral inflow. The most representative technique is the Ritter method, which estimates the optimal AVD based on the measured interval from the QRS onset to the end of A wave (active filling). Some variants of the Ritter method have also been proposed. Alternatively, the optimal AVD can be estimated, by maximizing the velocity time integral (VTI) of the aortic outflow or the mitral inflow. In addition, other Doppler-based methods for AVD optimization have also been explored, based on estimation of the cardiac output, the LV pressure derivative dP/dt, and the derived myocardial performance index (MPI), which is defined as the ratio of isovolumic contraction time plus the isovolumic relaxation time to the ejection time. Another non-invasive method for assessment of cardiac output is the thoracic impedance cardiography, which has been used for optimizing the AVD, and was found to give similar results as echocardiography. Recently, finger photoplethysmography as a simple method for non-invasive blood pressure monitoring, has been shown to be another attractive tool for optimizing AVD in cardiac resynchronization therapy (CRT) devices.

Alternatively, the AVD can be optimized based on hemodynamic indexes that are assumed to correlate to the stroke volume or cardiac output, such as the blood pressure or its temporal derivative, the ventricular volume (e.g., through chamber impedance measurement), the blood oxygen saturation, blood pH, blood temperature, etc.

Echo based CRT optimization is time consuming and requires patient compliance.

Electrogram-based CRT optimization may not be reliable since the metrics extracted from the ECG/EGM may not correlate well with the hemodynamic performance.

Many CRT optimization methods based on hemodynamic measurement require special sensors.

Types of CRT devices are CRT-P (CRT with pacemaker function) and CRT-D (CRT with pacemaker and ICD function).

US20130053912A1 discloses CRT optimization using impedance measurement of a multipolar LV lead.

US2005182447A1 teaches implantable cardiac devices such as pacemakers and defibrillators that deliver cardiac resynchronization therapy (CRT), and a method of optimizing acquisition of impedance signals between electrodes present on implanted lead systems.

US20100004712A1 describes techniques for detecting heart failure or other medical conditions within a patient using an implantable medical device, such as pacemaker or implantable cardioverter/defibrillator, or external system.

US8600497B1 teaches an implantable device that monitors and treats heart failure, pulmonary edema, and hemodynamic conditions and in some cases applies therapy.

US2012203090A1 describes techniques for use with an implantable medical device for assessing stroke volume or related cardiac function parameters such as cardiac output based on impedance signals obtained using hybrid impedance configurations that exploit a multi-pole cardiac pacing/sensing lead implanted near the left ventricle.

The invention is defined in the appended claims. Aspects, embodiments and examples disclosed of the present disclosure which do not fall under the scope of the appended claims do not form part of the invention and are merely provided for illustrative purposes.

It is an object of various embodiments of the invention to provide a heart stimulator comprising a stimulation control unit and one or more stimulation units, that can determine beneficial pacing parameters.

This object is achieved by means of a heart stimulator having an electric input that is connected or can be connected to a plurality of electrodes. The heart stimulator comprises a stimulation control unit, one or more stimulation units, an impedance determination unit and an impedance evaluation unit. The one or more stimulation units are configured to generate stimulation pulses and to deliver a stimulation pulse when triggered by said stimulation control unit. The stimulation control unit is operatively connected to the one or more stimulation units to control pacing parameters such as pacing site and/or the timing of stimulation pulses to be delivered by said one or more stimulation units. The impedance determination unit is configured to determine impedance values reflecting intracardiac impedance, and the impedance evaluation unit is configured to evaluate the impedance values and to provide an evaluated impedance signal to the stimulation control unit.

The impedance determination unit is configured to determine a plurality of impedance values between different pairs of electrodes that are connected to the electric input during operation of the heart stimulator. The impedance evaluation unit is configured to generate a mathematical combination of this plurality of impedance values. The mathematical combination is a sum, a weighted sum, a product, a weighted product or combinations thereof, of multiple impedance vectors derived from impedance values between different pairs of electrodes connected to said electric input during operation of the heart stimulator. The result of the mathematical combination is referred to as the composed impedance signal. The stimulation control unit is configured to determine respective pacing parameters such as pacing sites and/or timing of stimulation pulses depending on the composed impedance signal and/or the mathematical combination.

Various embodiments use a new parameter for CRT optimization in the context of CRT-P or CRT-D devices. In such a device with a plurality of electrodes, a composed impedance signal from impedance measurements between the multiple electrodes can be used to create a single parameter that is used to calculate optimal parameters of stimulation.

The stimulation control unit preferably is configured to determine a volume signal that reflects a change in heart volume between diastole and systole, wherein the volume signal is derived from a magnitude of the composed impedance signal and/or the mathematical combination of the impedance values during a respective heart cycle.

The composed impedance signal of impedance values preferably is used for optimization of pacing parameters so as to achieve maximum change in volume between diastole and systole and therefore maximize cardiac output.

Preferably, the stimulation control unit is configured to determine respective pacing sites and/or timing of stimulation pulses depending on the volume signal such that the pacing sites and the timing of stimulation pulses lead to maximum change in volume between diastole and systole and therefore maximize cardiac output.

With a composed impedance signal of a plurality of impedance values a drawback of conventional devices is overcome. Conventionally, CRT optimization based on impedance measurement relies on a single impedance vector, which covers only a fixed region of the heart with limited volume thus may not reflect the global cardiac volumetric change in systolic and diastolic phases of the myocardial contraction.

Preferably, the impedance determination unit is configured to determine impedance values via pairs of electrodes such that the area encompassed by the impedance vectors is maximized. When drawing the areas created by multiple vectors that correspond to various possible electrode pairs it occurs that the areas are different. It is preferred to use those electrode pairs that provide for the largest area. Thus measurements between RV and a superior placed LV electrode can be combined with an RV to inferiorly placed LV electrode to create a large triangle between them that encompasses a large 2-D area of the ventricle. All electrode pairs would be chosen with this in mind, to maximize the total area encompassed.

The impedance evaluation unit may be configured to perform combinations of the impedance values as impedance vectors in a geometric manner and combinations of the impedance vectors in a geometric manner that exceeds a simple weighted average, in fact, in order to determine the impedance across the area. This may also include measurement of impedance values from electrode to electrode on a multipolar LV lead. However, the same technique of creating impedance measurement planes can be applied to a unipolar LV lead. The key concept is measurement of impedance over an area via computation of the different impedance vectors.

While a single pair of electrodes would encompass only the impedance between two electrodes it is an advantage of a preferred embodiment that the area among 3 or more electrodes can create a plane that more adequately encompasses the 3-D volume and gives a better response in a heart that is twisting/compressing during the measurement cycle.

Preferably, the impedance determination unit is configured to measure impedance values representing left ventricular impedance across the ventricles while simultaneously or nearly simultaneously measuring impedance values representing right ventricular impedance as surrogate for stroke volume, and wherein the impedance evaluation unit is configured to remove the impedance values representing right ventricular impedance from an impedance vector or a combination of impedance vectors which encompasses impedance values representing both left and right ventricular impedance in order to select the impedance changes that are due to left ventricular stroke volume.

In a preferred embodiment, the impedance determination unit together with the impedance evaluation unit is configured to determine the electrodes used for impedance determination automatically.

The impedance vectors used to calculate the composed impedance signal, for example for the weighted sum are preferably those that maximize the area encompassed, depending upon the patient. The strength of the technique is to reduce the dependence upon the individual lead placement, as any set of electrodes may encompass a larger area in one patient but a small one in another. With a programmable system, it is possible to determine the appropriate combination in real time in a follow up setting or even in the active implant. In determining the ideal combination of electrodes, the three-dimensional area encompassed can also be considered.

The weighting factors for determining the weighted sum can be determined empirically on an individual or population basis as one example.

The weighting factors for determining the weighted sum can also be determined by calculating the area inside the multi vector combination (area of the triangle created by three electrodes or even a rectangle created by four electrodes). In the case of a rectangle approximation, this would be for example Z_RVtip to Z_RVcoil multiplied by Z_RVtip to Z_LV1

Preferably, the impedance evaluation unit is configured to determine the time course of the impedance area over time and to use the minimum and the maximum of the time course of the impedance area to determine relative stroke volume.

Alternatively or additionally, the impedance evaluation is configured to determine a maximum of the change of the composed impedance signal like the weighted sum over time (dZ/dt) or other properties of the waveform, for instance 90% maximum. In addition, as the impedance evaluation unit preferably calculates an area from the multiple electrodes, it is further preferred if the impedance evaluation unit is configured to calculate the area under the curve when considering the area over time. The area calculation would be plotted versus time and the area under that curve would be calculated/integrated to determine total stroke volume change.

According to a further preferred embodiment, the impedance evaluation unit is configured to evaluate the time relation of the individual impedance vectors.

The proposed method preferably utilizes measurement of impedance from a multipolar lead. By combining appropriately the signal from multiple vectors simultaneously to a composed signal, the impedance measurement covers a larger volume of the heart and is not focused on the field between a single electrode pair. This combination can provide an improved measurement of cardiac volume changes between systole and diastole, allowing more specific measurement of volume and more efficacious optimization of pace timing. This provides a competitive advantage over current methods of optimization in terms of accuracy while allowing real-time adjustment of parameters if desired, rather than optimization only in an office setting as is currently available from prior art devices.

In a preferred embodiment, the impedance evaluation unit is configured to calculate the mathematical combination as a weighted sum. Alternatively, the impedance evaluation unit is configured to calculate said mathematical combination as vector combination of impedance vectors or to calculate said mathematical combination as product of impedance vectors.

Preferably, the impedance evaluation unit is configured to calculate an area inside the vector combination of impedance vectors by multiplying the impedance vector between a first pair of electrodes by the impedance vector between a second pair of electrodes.

Alternatively or additionally, the impedance evaluation unit is configured to determine weighting factors for determining a weighted sum by the area inside the vector combination of impedance vectors or to determine a time course of the area inside the vector combination of impedance vectors and to use the minimum and the maximum of the time course of the area to determine relative stroke volume.

In a preferred embodiment, the impedance evaluation unit is configured to determine a maximum of the change of the mathematical combination of the impedance values over time. Alternatively, the impedance evaluation unit is configured to calculate the area under a curve describing the change of impedance values over time and to evaluate the time course of the area over time.

The above and other aspects, features and advantages of the present invention will be more apparent from the following more particular description thereof, presented in conjunction with the following drawings wherein:
- Figure 1: shows a three chamber bi-ventricular implantable cardioverter/defibrillator (ICD).
- Figures 2 and 3: are schematic diagrams of three alternative embodiments of the device modules of the pacemaker/ICD of figure 1.
- Figure 4a: shows a typical electrocardiogram.
- Figure 4b: depicts a typical time course of the left ventricular impedance Z.
- Figures 5 A and B: illustrate two different impedance measurement vectors.
- Figure 6: illustrates a number of possible impedance measurement vectors if the left ventricular electrode lead features four electrodes.

The following description is of the best mode presently contemplated for carrying out the invention. This description is not to be taken in a limiting sense, but is made merely for the purpose of describing the general principles of the invention. The scope of the invention should be determined with reference to the claims.

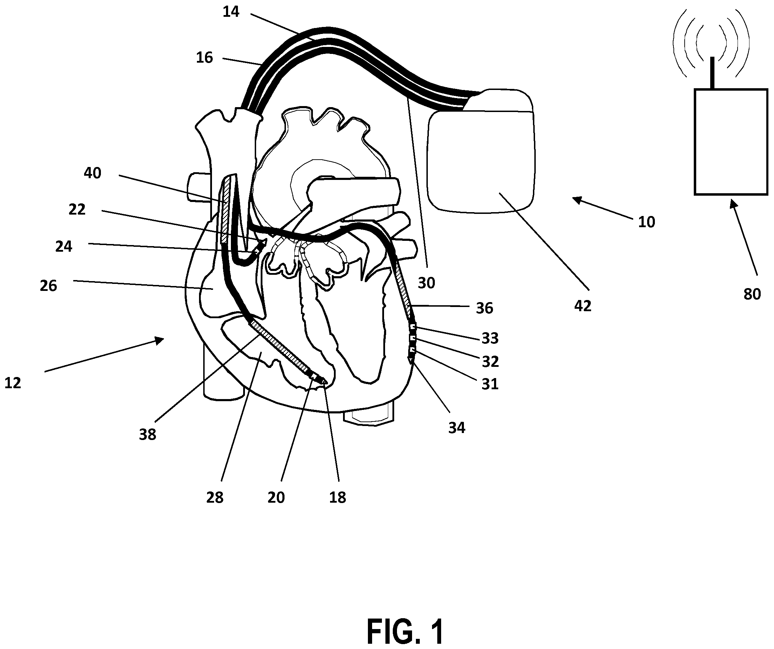

In figure 1 the implantable medical device (also referred to as implantable cardiac device) is a three chamber biventricular pacemaker and cardioverter/defibrillator 10 that is connected to pacing/sensing leads placed in a heart 12 is illustrated.

As shown in figure 1, the preferred embodiment is to couple the disclosed technology with an implantable medical device like an implantable bi-ventricular cardioverter/defibrillator (ICD). The embodiments are also applicable to a pacemaker.

The implantable medical device 10 is electrically coupled to heart 12 by way of leads 14, 16 and 30.

Lead 14 is a right atrial electrode lead that has a pair of right atrial electrodes 22 and 24 that are in contact with the right atria 26 of the heart 12.

Lead 16 is a right ventricular electrode lead that has a pair of ventricular stimulation and sensing electrodes 18 and 20 that are in contact with the right ventricle 28 of heart 12. Further, a ventricular defibrillation shock coil 38 and an atrial defibrillation shock coil 40 are arranged on lead 16.

Electrodes 22 and 18 are tip electrodes at the very distal end of leads 14 and 16, respectively. Electrode 22 is a right atrial tip electrode RA Tip and electrode 18 is a right ventricular tip electrode. Electrodes 24 and 20 are ring electrodes in close proximity but electrically isolated from the respective tip electrodes 22 and 18. Electrode 24 forms a right atrial ring electrode RA Ring and electrode 20 forms a right ventricular ring electrode RV Ring. Atrial cardioversion shock coil 40 is a coil electrode providing a relatively large geometric area when compared to the stimulation electrodes 18, 20, 22 and 24.

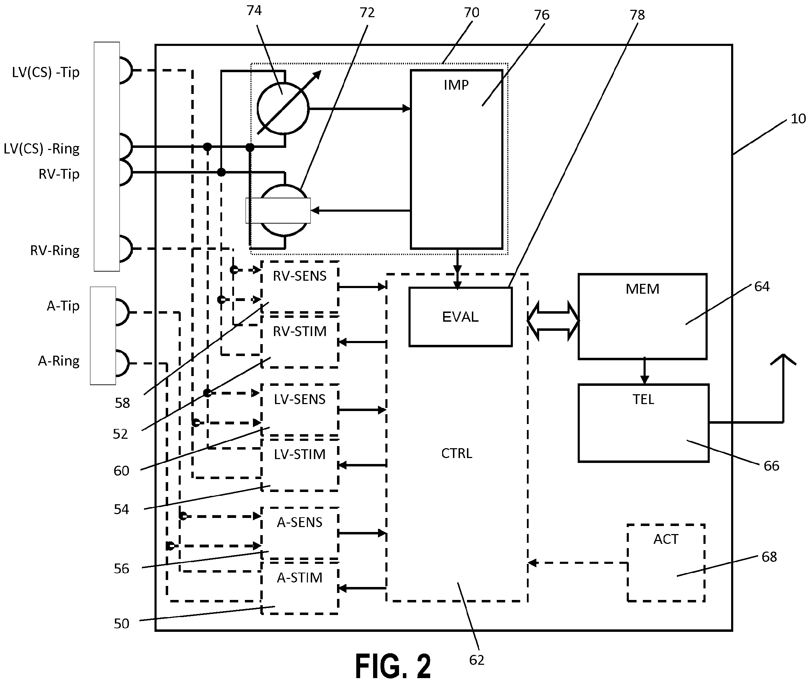

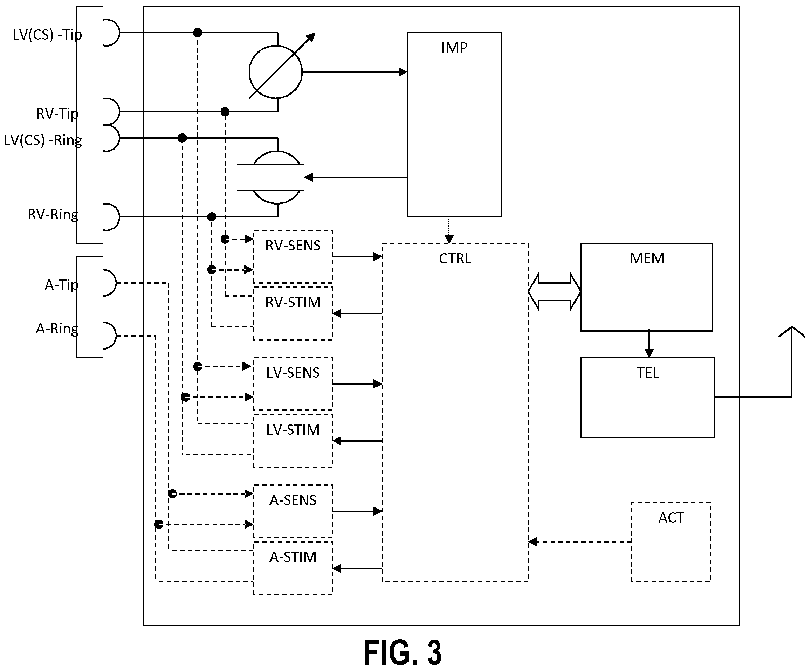

Lead 30 is a left ventricular electrode lead passing through the coronary sinus of heart 12 and having left ventricular ring electrodes LV RING 31, 32 and 33 and a left ventricular tip electrode LV TIP 34. Further, a left ventricular defibrillation shock coil 36 is arranged on lead 30. It is noted that the number of left ventricular ring electrodes may vary depending on the electrode lead that is used. In the context of figures 2 and 3, if one left ventricular ring electrode LV-RING is referred to, this can be one of the left ventricular ring electrodes 31, 32, 33 or the shock coil 36. In figures 2 and 3 LV-TIP is denominated as LV(CS)-Tip and LV-RING is denominated as LV(CS)-Ring.
Implantable medical device 10 has a case 42 made from electrically conductive material such as titanium that can serve as a large surface electrode IMD CASE.

The plurality of electrodes 18, 20, 22, 24, 31, 32, 33, 34, 36, 38 and 40 connected to implantable medical device 10 together with case 42 allow for a number of different electrode configurations for measuring intrathoracic and intracardiac impedance. In general, any combination of the plurality of electrodes 18, 20, 22, 24, 31, 32, 33, 34, 36, 38 and 40 and the case 42, wherein the case is considered to act as electrode, is possible for bipolar, tripolar or quadrupolar impedance measurement as known in the art.

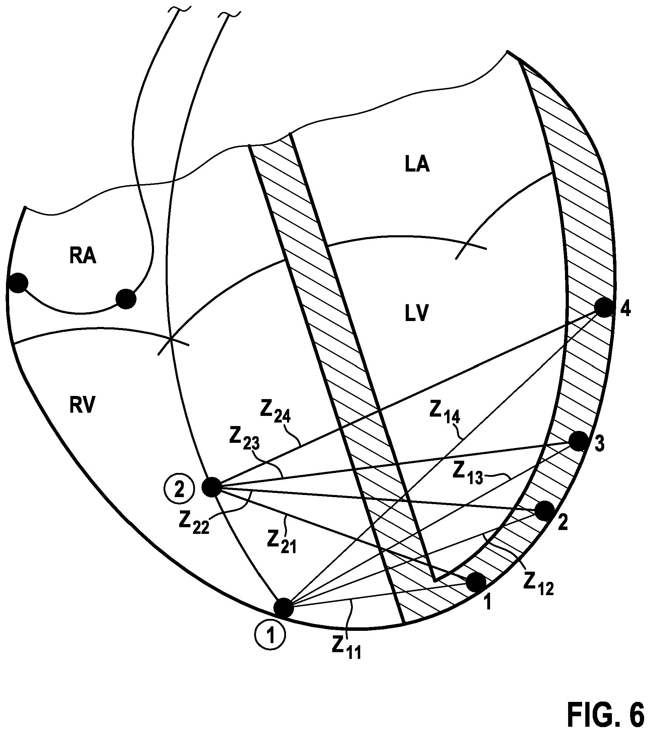

For each individual one of the intracardiac impedance measurements, injecting a forcing function from a first electrode, for example the right ventricular ring electrode to a second electrode, for example the left ventricular ring electrode and measuring a response function between the same electrodes (bipolar configuration; see figure 2) or, for instance, measuring a response function between a third electrode, for example the right ventricular tip electrode and a fourth electrode, for example the left ventricular tip electrode (quadrupolar configuration; see figure 3) is possible. Further possible impedance measurement vectors (resulting from different impedance measurement electrode combinations) are apparent to those skilled in the art and are exemplarily, but not limited to illustrated with respect to figure 6.

Referring to figure 2 illustrating a simplified block diagram of an implantable medical device 10. During operation of the pacemaker leads 14, 16 and 30 are connected to respective output/input terminals of pacemaker 10 as indicated in figure 1 and carry stimulating pulses to the atrial tip electrode A-Tip 22 and the atrial ring electrode A-Ring 24 from a right atrial stimulation pulse generator A-STIM 50; to the right ventricular tip electrode RV-Tip 18 and the right ventricular ring electrode RV-Ring 20 from a right ventricular pulse generator RV-STIM 52 and to a left ventricular tip electrode LV(CS)-Tip 34 and one of the left ventricular ring electrodes LV(CS)-Ring 31, 32, 33, 36 from a left ventricular pulse generator LV-STIM 54, respectively. This exemplary pacing configuration is known as bipolar pacing configuration. One output of the stimulation pulse generators may be alternatively connected to the case to achieve the also known unipolar pacing configuration. Further, electrical signals from the right atrium are carried from the electrode pair 22 and 24, through the lead 14, to the input terminal of a right atrial channel sensing stage A-SENS 56; and electrical signals from the right ventricle are carried from the electrode pair 18 and 20, through the lead 16, to the input terminal of a right ventricular sensing stage RV-SENS 58. Likewise electrical signals from the left ventricle are carried from the electrode pair 34 and one of 31, 32, 33, 36, through the lead 30, to the input terminal of a left ventricular sensing stage LV-SENS 60. This exemplary sensing configuration is known as bipolar sensing configuration. One input of the sensing stages may be alternatively connected to the case to achieve the also known unipolar sensing configuration.

The implantable medical device may comprise additional stimulation pulse generators and sensing stages (not shown in figure 2 and 3) connectable to the electrodes of the pacemaker leads 14, 16 and 30 enable additional pacing and sensing capabilities. For example if left ventricular electrodes 34 and 31 are connected to the left ventricular stimulation pulse generator LV-STIM 54 and left ventricular sensing stage LV-SENS 60, left ventricular electrodes 34 and 32 may be connected to a additional left ventricular stimulation pulse generator and an additional left ventricular sensing stage are (not shown in figure 2 and 3).

The implantable medical device 10 is controlled by a control unit CTRL 62 that is connected to sensing stages A-SENS 56, RV-SENS 58 and LV-SENS 60 and to stimulation pulse generators A-STIM 50, RV-STIM 52 and LV-STIM 54. Control unit CTRL 62 receives the output signals from the atrial sensing stage A-SENS 56, from the right ventricular sensing stage RV-SENS 58 and from the left ventricular sensing stage LV-SENS 60. The output signals of sensing stages A-SENS 56, RV-SENS 58 and LV-SENS 60 are generated each time that a P-wave representing an intrinsic atrial event or an R-wave representing an intrinsic ventricular event, respectively, is sensed within the heart 12. An As-signal is generated, when the atrial sensing stage A-SENS 56 detects a P-wave and a RVs-signal is generated, when the right ventricular sensing stage RV-SENS 58 detects an R-wave.

These sense events are used by control unit CTRL 62 as fiducial points of the respective intracardiac electrograms picked up by the sensing stages A-SENS 56, RV-SENS 58 and LV-SENS 60, respectively.

Control unit CTRL 62 also generates trigger signals that are sent to the atrial stimulation pulse generator A-STIM 50, the right ventricular stimulation pulse generator RV-STIM 52 and the left ventricular stimulation pulse generator LV-STIM 54, respectively. These trigger signals are generated each time that a stimulation pulse is to be generated by the respective pulse generator A-STIM 50, RV-STIM 52 or LV-STIM 54. The atrial trigger signal is referred to simply as the "A-pulse", and the ventricular trigger signal is referred to as the "RV-pulse" or the "LV-pulse", respectively. During the time that either an atrial stimulation pulse or ventricular stimulation pulse is being delivered to the heart, the corresponding sensing stage, A-SENS 56, RV-SENS 58 and/or LV-SENS 60, is typically disabled by way of a blanking signal presented to these amplifiers from the control unit CTRL 62, respectively. This blanking action prevents the sensing stages A-SENS 56, RV-SENS 58 and LV-SENS 60 from becoming saturated from the relatively large stimulation pulses that are present at their input terminals during this time. This blanking action also helps prevent residual electrical signals present in the muscle tissue as a result of a stimulation pulse delivered from pacemaker 10 from being interpreted as P-waves or R-waves.

Furthermore, atrial sense events As recorded shortly after delivery of a ventricular stimulation pulses during a preset time interval called post ventricular atrial refractory period (PVARP) are generally recorded as atrial refractory sense event Ars but ignored.

Control unit CTRL 62 comprises circuitry for timing ventricular and/or atrial stimulation pulses according to an adequate stimulation rate that can be adapted to a patient's hemodynamic need as pointed out below.

Still referring to figure 2, the implantable medical device 10 includes a memory circuit MEM 64 that is coupled to the control unit CTRL 62 over a suitable data/address bus ADR. This memory circuit MEM 64 allows certain control parameters, used by the control unit CTRL 62 in controlling the operation of the implantable medical device 10, to be programmably stored and modified, as required, in order to customize the implantable medical device's operation to suit the needs of a particular patient. Such data includes the basic timing intervals used during operation of the pacemaker 10 and AV delay values and hysteresis AV delay values in particular.

Further, data sensed during the operation of the implantable medical device 10 may be stored in the memory MEM 64 for later retrieval and analysis.

A telemetry circuit TEL 66 is further included in the implantable medical device 10. This telemetry circuit TEL 6 is connected to the control unit CTRL 62 by way of a suitable command/data bus. Telemetry circuit TEL 66 allows for wireless data exchange between the implantable medical device 10 and some remote programming or analyzing device which can be part of a centralized service center serving multiple pacemakers.

The implantable medical device 10 in figure 2 is referred to as a three chamber pacemaker/cardioverter/defibrillator because it interfaces with the right atrium 26, the right ventricle 28 and the left ventricle of the heart 12. Those portions of the pacemaker 10 that interface with the right atrium, e.g., the lead 14, the P-wave sensing stage A-SENSE 56, the atrial stimulation pulse generator A-STIM 50 and corresponding portions of the control unit CTRL 62, are commonly referred to as the atrial channel. Similarly, those portions of the pacemaker 10 that interface with the right ventricle 28, e.g., the lead 16, the R-wave sensing stage RV-SENSE 58, the ventricular stimulation pulse generator RV-STIM 52, and corresponding portions of the control unit CTRL 62, are commonly referred to as the ventricular channel.

In order to be able to detect periods of physical activity of a patient indicating that the patient is awake and in order to allow rate adaptive pacing in a DDDR or a DDIR mode, the pacemaker 10 further includes a physiological sensor ACT 68 that is connected to the control unit CTRL 62 of the pacemaker 10. While this sensor ACT 68 is illustrated in figure 2 as being included within the pacemaker 10, it is to be understood that the sensor may also be external to the implantable medical device 10, yet still be implanted within or carried by the patient. A common type of sensor is an accelerometer, such as a piezoelectric crystal, mounted to the case of the pacemaker. Other types of physiologic sensors are also known, such as sensors that sense the oxygen content of blood, respiration rate, blood pH, intracardiac impedance changes, and the like. The type of sensor used is not critical. Any sensor capable of sensing some physiological parameter relatable to physical activity of a patient can be used. Such sensors are commonly used with "rate-responsive" pacemakers in order to adjust the rate of the pacemaker in a manner that tracks the physiological needs of the patient. The output of sensor 68 represents an activity level.

By means of the output signal of activity sensor 68 the control unit 62 is able to assign each intrinsic heart rate to an activity thus enabling collection of intrinsic heart rate value for a patient's state of rest and a patient's state of exercise separately.

The control unit CTRL 62 is adapted to determine an adequate heart rate or stimulation rate in any manner known as such.

For impedance measurement, an impedance determination unit 70 is provided. Impedance determination unit 70 comprises a constant current source 72 that is connected or can be connected to electrodes for intracorporeal placement as shown in figure 1. In order to allow for a plurality of impedance measurement electrode configurations, preferably some means of switching is provided between the constant current source 72 and the electrode terminals of the implantable medical device 10. The switch is not shown in figure 2. Rather, particular impedance measurement configurations are shown as examples.

Similarly, a voltage measuring unit 74 for measuring a voltage corresponding to a current fed through a body by said constant current source is provided and can be connected to a number of electrodes although a switch for switching between these configurations is not shown in figure 2.

As an alternative to constant current source 72 a constant voltage source can be provided to generate the forcing function. Then, the measuring unit will be adapted to measure a current strength of a current fed through a body by said constant voltage source.

Both, constant current source 72 and voltage measurement unit 74, are connected to an impedance value determination unit 76 that is adapted to determine an impedance value for each measuring current pulse delivered by the constant current source 72.

Further, an impedance evaluation unit 78 is provided either as a separate unit or as part of control unit CTRL 62 as depicted in Figure 2. The evaluation unit 78 is connected to said impedance measurement unit 70 and is adapted to evaluate a sequence of consecutive impedance values determined by said impedance measurement unit. The evaluation unit 78 comprises a signal generator module (not shown) to construct the intracardiac impedance or conductance signal reflecting the time course of the impedance measurement unit's output signal and its derivative.

The evaluation unit 78 further comprises a filter module (not shown) to filter the intracardiac impedance signal.

The evaluation unit 78 is further connected to the right ventricular stimulation stage RV-STIM 52 and the right ventricular sensing stage RV-SENS 58 in order to receive signals representing cardiac events, namely right ventricular stimulation events RVp or right ventricular sense events RVs, respectively.

The constant current source 72 has its two poles connected to different connectors for different electrodes as for example the right ventricular ring electrode and the left ventricular ring electrode (Fig. 2) or the left ventricular tip electrode and the right ventricular tip electrode (Fig. 3). The voltage measuring unit 74 has two poles connected to, for example, a connector for the left ventricular ring electrode and the right ventricular ring electrode (Fig. 2) or the left ventricular ring electrode and the right ventricular ring electrode (Fig. 3). Thus, a bipolar or a quadrupolar impedance measurement configuration is established.

Impedance measurement is carried out by injecting a constant current and sampling the resulting voltage.

The measuring current is preferably pulsed. Typically, the measuring current will feature biphasic pulses wherein two constant current pulses of opposite polarity form one pulse package. Two consecutive pulse packages between two consecutive pulse packages a time gap is provided, which is significantly longer than the duration of one pulse package. The constant current pulses within one pulse package are each of the same intensity and of same duration. They only have different polarities. The typical value for the intensity of the constant current pulses is between 50 µA and 600 µA. The typical pulse duration of a single constant current pulse is about 15 µs.

The time gap between each two consecutive pulse packages may be 500 times longer than the duration of one constant current pulse. The two constant current pulses of opposite polarity within one pulse package may not follow immediately each other but may have a time gap there between. This time gap however, will be very short compared to the time gap between two consecutive pulse packages. Furthermore, consecutive pulse packages may be face alternating such that a first pulse package for example will begin with a positive constant current pulse whereas the following pulse package will begin with a negative constant current pulse and end with a positive constant current pulse.

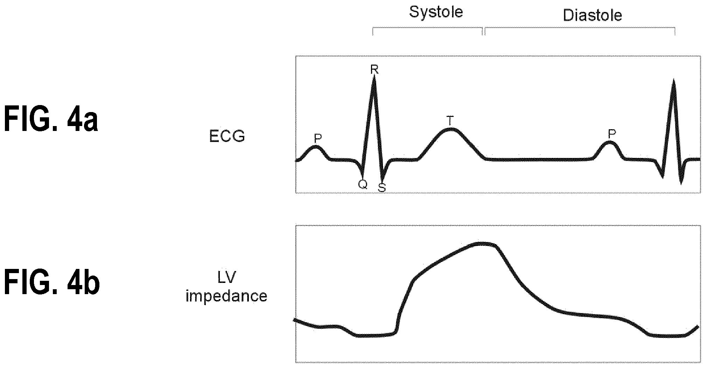

In Fig. 4b a typical time course of the left ventricular impedance Z is depicted. Fig. 4a shows a typical electrocardiogram. When the left ventricle has its smallest volume at the end of the systole (contraction of the ventricle) the impedance Z has a maximum. The time course of the impedance inversely reflects the time course left ventricular volume.

The main purpose of the sensing stages 56, 58 and 60 is to detect a natural (intrinsic) contraction of the respective heart chamber in order to generate a sense event signal like an atrial sense event As, a right ventricular sense event RVs and a left ventricular sense event LVs. These sense events are processed by the control unit CTRL 62 in order to inhibit a delivery of a stimulation pulse when the pacemaker is operating in a demand mode or in order to determine a time interval between an atrial event and a point of time, when the course of the left ventricular intracardiac impedance reaches its minimum value, see below.

Another type of event to be processed by the control unit CTRL 62 would be the delivery of a stimulation pulse to a respective heart chamber. Delivery of a stimulation pulse causes a paced event such as an atrial paced event Ap, a right ventricular paced event RVp and a left ventricular paced event LVp.

According to a preferred embodiment, different optimal time delays for different stimulation (pacing) rates and thus for different states of metabolic demand are determined. Therefore, memory MEM 64 is provided and connected to control unit CTRL 62 which is adapted to store optimal time delays such as optimal AV-delays AVD_opt and optimal VV-delays VVD_opt for different states of exertion.

Memory MEM 64 also serves for storing transient values for measured impedances (for example end systolic impedance ESZ) and tested time delays associated therewith as disclosed in more detail further below.

The impedance evaluation unit EVAL 78 is adapted to determine from the time course of the impedance value time periods for each cardiac cycle that correspond to a filling period, an isovolumic contraction period, an ejection period and an isovolumic relaxation period, respectively.

When in use, the implantable cardiac device measures the intracardiac impedance (Z), that may be a composed intracardiac impedance, from which a plurality of timing intervals are derived that characterize different phases of the cardiac contraction, such as the isovolumic contraction time (IVCT), isovolumic relaxation time (IVRT), ejection time (ET), and filling time (FT). The total isovolumic time (TIVT) is the sum of IVCT and IVRT, and the cardiac cycle length (CL) is the sum of IVCT, IVRT, ET, and FT. At least three metrics could be derived from these timing intervals. The Systolic Performance Index (SPI) is defined as the ratio of TIVT to ET. The Diastolic Performance Index (DPI) is defined as the ratio of TIVT to FT. The Cardiac Performance Index (CPI) is defined as the ratio of TIVT to CL. According to a preferred embodiment, the pacing parameters, including the A-V delay (AVD), V-V delay (VVD), and the pacing site (for example, to choose the optimal LV pacing vector using a multi-polar LV electrodes), are considered optimal when the setting results in minimum CPI. In another embodiment, the pacing parameters are considered to be optimal when the setting results in minimum SPI or minimum DPI.

The intracardiac impedance (Z) can be measured in various means. Preferably, the impedance vectors span across the left ventricle (LV) to reflect (at least) the volume change of the left ventricle.

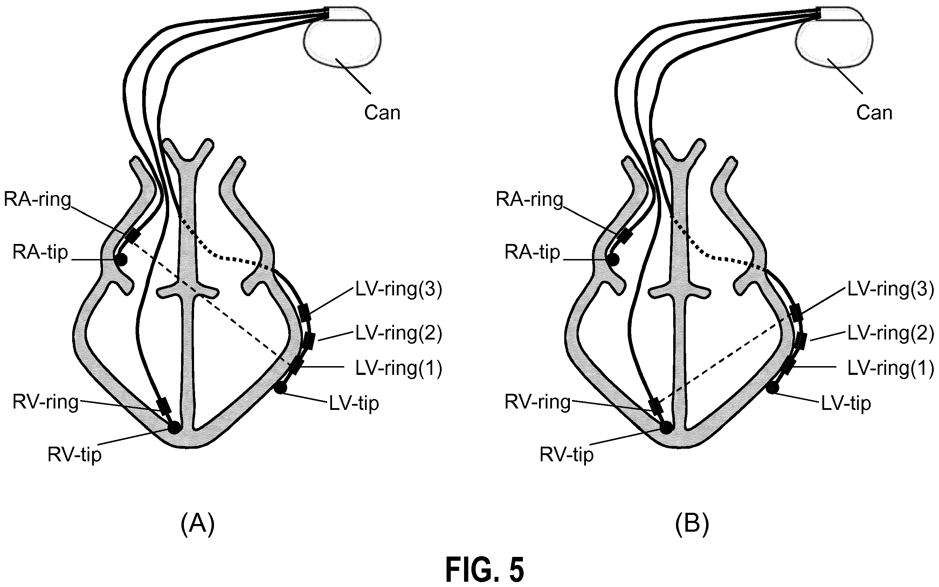

Figure 5 shows two typical examples. In Figure 5A, subthreshold biphasic current pulses are injected between the RA tip and LV tip electrodes, and the voltage is measured between the RA ring and LV ring electrodes. The ratio of the measured voltage to the injected current then approximately represents the impedance between the distal end of the RA lead and the distal end of the LV lead. In Figure 5B, subthreshold biphasic current pulses are injected between the RV tip and LV tip electrodes, and the voltage is measured between the RV ring and LV ring electrodes. The ratio of the measured voltage to the injected current then approximately represents the impedance between the distal end of the RV lead and the distal end of the LV lead. In both examples, the impedance vector passes through the LV chamber, thus the measured impedance is affected by the blood volume change of the LV. Clearly, it should be understood that LV impedance can also be measured by other electrode configurations, and that the different measurements can be combined in a weighted sum that serves a single parameter that is used by control unit 62 to determine optimal parameters of stimulation. Selection of various electrode configurations and thus a switching between different impedance measurements vectors can be achieved by means of switch matrix (not shown) that is arranged between impedance determination unit 70 and input terminals that are connected to individual electrodes. The switch matrix can be controlled by either impedance determination unit 70 or control unit 62.

In this respect, it is to be noted, that besides AV/VV delay optimization, the disclosed concept is also applicable to pacing site optimization. Specifically, when a multi-polar LV lead is used (as in figures 5A, 5B and 6), the LV pacing site can be optimized by choosing the LV electrode (thus the LV pacing vector) that maximizes the changes of the composed impedance signal like the weighted sum of the impedance values over one heart cycle. Due to the anatomic constraint of the coronary venous, conventional unipolar or bipolar LV lead may not be able to pace the myocardial region with the most delayed activation or avoid the infarct area. Employing multipolar LV lead, in conjunction with the proposed optimization method, it is feasible to select the optimal LV pacing site that improves the intraventricular synchrony and maximizes the cardiac performance.

Reference is now made to figure 6, which illustrates a plurality of impedance measurement vectors that can be used to obtain a composed impedance as a parameter for CRT optimization in the context of CRT-P or CRT-D devices. In such a device with a plurality of electrodes, the composed impedance signal like a weighted sum of impedance measurements between the multiple electrodes can be used to create a single parameter that is used to calculate optimal parameters of stimulation. Stimulation parameters may include Stimulation amplitude, duration, timing between Stimulation pulse and intrinsic events or between a Stimulation pulse on one electrode and another (for instance between multiple electrodes within the heart). Stimulation parameters also include selection of a stimulation electrode which is being used or which combination of electrodes is being used and the timing between those used stimulation electrodes.

The weighted sum of impedances includes for instance the sum of Wll * Z11+W12 * Z12+W13 * Z13+W14 * Z14 as a Single parameter where Wn,m represents the weight and Zn,m represents the impedance measured between individual electrode pairs "n" and "m", as shown in Figure 6. This weighted sum may be extended to any combination of electrodes either in the heart or in the body in general. The weighted sum can be created in a way to maximize the total 2-D area or 3-D volume that is contributing to the measured sum in order to use the weighted sum as a surrogate for volume within any one or multiple heart chambers.

The composed impedance and/or the mathematical combination in an alternative embodiment is calculated as vector combination of a plurality of impedance vectors.
The composed impedance and/or the mathematical combination in yet another alternative embodiment is calculated as product of impedance vectors. The impedance vectors may be weighted.

Alternatively, the impedance evaluation unit is configured to determine weighting factors for determining a weighted sum by calculating an area inside a multi impedance measurement vector combination by multiplying the impedance measurement between a first pair of electrodes by the impedance measurement between a second pair of electrodes.

Changes in the parameter such as peak to peak variation, total area under the curve, rate of change over time, or other extracted measures may be used to indicate changes over long or short term including posture changes, hemodynamic changes, remodeling or pathologic changes in the heart or any process which causes a change in the parameter.

The composed impedance parameter may also be utilized within a closed loop system where the parameter is held constant over time by varying the before mentioned Stimulation parameters. For instance, timing between left and right ventricular activation is varied to achieve a stable and constant value of the composed impedance, for example the weighted sum impedance parameter. Changes in impedance between one set of electrodes (i.e. on left side of heart) may be used as statistical controls in order to determine the true variation in impedance between other pairs of electrodes (i.e. between right and left sides of the heart). In its simplest form this could mean subtracting variations measured within the left side of the heart from the variations measured across the heart in order to remove the near-field impedance changes from the far-field (left to right side) changes.

Generally, a decrease in the impedance signal implies increase in blood volume along the measurement vector, for example, during ventricular filling, whereas an increase in the impedance signal correlates to decrease in blood volume along the measurement vector, for example, during ventricular systole.

According to varioius embodiments, the LV impedance signal is recorded by varying different pacing parameters, including the A-V delay (AVD), V-V delay (VVD), and the pacing site. For each configuration, the composed impedance is determined.

The heart stimulator and the method described herein provide a means of optimizing pacing parameters based on a single parameter derived from a plurality of impedance measurements. The optimization scheme can be automated in an implantable cardiac device. The optimization strategy can be individually tailored to optimize the overall cardiac function. It allows frequent or periodic optimization of the pacing parameters under different load conditions, and does not require patient for a follow-up visit.

## Claims

1. A heart stimulator (10) having an electric input that is connected to a plurality of electrodes (18, 20, 22, 24, 31, 32, 33, 34, 36, 38, 40), said heart stimulator comprising a stimulation control unit (62), one or more stimulation units (50, 52, 54), an impedance determination unit (70) and an impedance evaluation unit (78),
said one or more stimulation units (50, 52, 54) being configured to generate stimulation pulses and to deliver a stimulation pulse when triggered by said stimulation control unit (62),
said stimulation control unit (62) being operatively connected to said one or more stimulation units (50, 52, 54) to control parameters such as pacing site and/or timing of stimulation pulses to be delivered by said one or more stimulation units (50, 52, 54),
said impedance determination unit (70) being configured to determine impedance values reflecting intracardiac impedance, and
said impedance evaluation unit (78) being configured to evaluate said impedance values and to provide an evaluated impedance signal to said stimulation control unit (62),
wherein said impedance determination unit (70) is configured to determine a plurality of impedance values between different pairs of electrodes (18, 20, 22, 24, 31, 32, 33, 34, 36, 38, 40) connected to said electric input and wherein said impedance evaluation unit (78) is configured to generate a mathematical combination of said impedance values between different pairs of electrodes connected to said electric input and wherein said stimulation control unit (62) is configured to determine respective pacing sites and/or timing of stimulation pulses depending on said mathematical combination,
**characterized in that**
the impedance evaluation unit (78) is configured to generate said mathematical combination of said impedance values as impedance vectors in a geometric manner, wherein the impedance determination unit (70) is configured to determine impedance values via pairs of electrodes such that the area encompassed by the respective impedance vectors is maximized.

2. The heart stimulator of claim 1, wherein said stimulation control unit (62) is configured to determine a volume signal that reflects a change in heart volume between diastole and systole, wherein said volume signal is derived from a magnitude of the mathematical combination of said impedance values during a respective heart cycle.

3. The heart stimulator of claim 2, wherein said stimulation control unit (62) is configured to determine respective pacing sites and/or timing of stimulation pulses depending on said volume signal such that the pacing sites and the timing of stimulation pulses lead to maximum change in volume between diastole and systole and therefore maximize cardiac output.

4. The heart stimulator of claim 1, wherein the impedance determination unit (70) is configured to measure impedance values representing left ventricular impedance across the ventricles while simultaneously measuring impedance values representing right ventricular impedance as surrogate for stroke volume, and wherein the impedance evaluation unit (78) is configured to remove the impedance values representing right ventricular impedance from an impedance vector or a combination of impedance vectors which encompasses impedance values representing both left and right ventricular impedance in order to select the impedance changes that are due to left ventricular stroke volume.

5. The heart stimulator of claim 1, wherein the impedance determination unit (70) together with the impedance evaluation unit (78) is configured to determine the electrodes used for determination of the impedance values automatically.

6. The heart stimulator of claim 1, wherein the impedance evaluation unit (78) is configured to calculate said mathematical combination as a weighted sum.

7. The heart stimulator of claim 1, wherein the impedance evaluation unit (78) is configured to calculate said mathematical combination as vector combination of impedance vectors or to calculate said mathematical combination as product of impedance vectors.

8. The heart stimulator of claim 7, wherein the impedance evaluation unit (78) is configured to calculate an area inside the vector combination of impedance vectors by multiplying the impedance vector between a first pair of electrodes by the impedance vector between a second pair of electrodes.

9. The heart stimulator of claim 8, wherein the impedance evaluation unit (78) is configured to determine weighting factors for determining a weighted sum by the area inside the vector combination of impedance vectors or to determine a time course of the area inside the vector combination of impedance vectors and to use the minimum and the maximum of the time course of the area to determine relative stroke volume.

10. The heart stimulator of claim 1, wherein the impedance evaluation unit (78) is configured to determine a maximum of the change of the mathematical combination of the impedance values over time.

11. The heart stimulator of claim 7 to 9, wherein the impedance evaluation unit (78) is configured to calculate the area under a curve describing the change of impedance over time and to evaluate the time course of the area over time.

12. The heart stimulator of claim 1, wherein the impedance evaluation unit (78) is configured to evaluate the time relation of the individual impedance vectors.

## Patentansprüche

1. Herzstimulator (10) mit einem elektrischen Eingang, der mit mehreren Elektroden (18, 20, 22, 24, 31, 32, 33, 34, 36, 38, 40) verbunden ist, wobei der genannte Herzstimulator eine Stimulationssteuereinheit (62), eine oder mehr Stimulationseinheiten (50, 52, 54), eine Impedanzbestimmungseinheit (70) und eine Impedanzauswertungseinheit (78) aufweist,
wobei die genannte(n) eine oder mehr Stimulationseinheiten (50, 52, 54) konfiguriert sind zum Erzeugen von Stimulationsimpulsen und zum Abgeben eines Stimulationsimpulses bei Auslösung durch die genannte Stimulationssteuereinheit (62),
wobei die genannte Stimulationssteuereinheit (62) funktionell verbunden ist mit der/den genannten einen oder mehr Stimulationseinheiten (50, 52, 54) zum Steuern von Parametern wie Stimulationsort und/oder zeitliche Steuerung von Stimulationsimpulsen, die durch die genannte(n) eine oder mehr Stimulationseinheiten (50, 52, 54) abzugeben sind,
wobei die genannte Impedanzbestimmungseinheit (70) konfiguriert ist zum Bestimmen von intrakardiale Impedanz repräsentierenden Impedanzwerten und wobei die genannte Impedanzauswertungseinheit (78) konfiguriert ist zum Auswerten der genannten Impedanzwerte und zum Anlegen eines ausgewerteten Impedanzsignals an die genannte Stimulationssteuereinheit (62),
wobei die genannte Impedanzbestimmungseinheit (70) konfiguriert ist zum Bestimmen von mehreren Impedanzwerten zwischen verschiedenen Paaren von Elektroden (18, 20, 22, 24, 31, 32, 33, 34, 36, 38, 40), die mit dem genannten elektrischen Eingang verbunden sind, und wobei die genannte Impedanzauswertungseinheit (78) konfiguriert ist zum Erzeugen einer mathematischen Kombination der genannten Impedanzwerte zwischen verschiedenen mit dem genannten elektrischen Eingang verbundenen Elektrodenpaaren und wobei die genannte Stimulationssteuereinheit (62) konfiguriert ist zum Bestimmen jeweiliger Stimulationsorte und/oder zeitlicher Steuerung von Stimulationsimpulsen in Abhängigkeit von der genannten mathematischen Kombination,
**dadurch gekennzeichnet, dass**
die Impedanzauswertungseinheit (78) konfiguriert ist zum Erzeugen der genannten mathematischen Kombination der genannten Impedanzwerte als Impedanzvektoren auf eine geometrische Weise, wobei die Impedanzbestimmungseinheit (70) konfiguriert ist zum Bestimmen von Impedanzwerten über Elektrodenpaare, so dass der von den jeweiligen Impedanzvektoren umschlossene Bereich maximiert wird.

2. Herzstimulator nach Anspruch 1, wobei die genannte Stimulationssteuereinheit (62) konfiguriert ist zum Bestimmen eines Volumensignals, das eine Änderung des Herzvolumens zwischen Diastole und Systole repräsentiert, wobei das genannte Volumensignal von einer Größe der mathematischen Kombination der genannten Impedanzwerte während eines jeweiligen Herzzyklus abgeleitet wird.

3. Herzstimulator nach Anspruch 2, wobei die genannte Stimulationssteuereinheit (62) konfiguriert ist zum Bestimmen jeweiliger Stimulationsorte und/oder zeitlicher Steuerung von Stimulationsimpulsen in Abhängigkeit von dem genannten Volumensignal, so dass die Stimulationsorte und die zeitliche Steuerung von Stimulationsimpulsen zu maximaler Änderung des Volumens zwischen Diastole und Systole führen und daher das Herzzeitvolumen maximieren.

4. Herzstimulator nach Anspruch 1, wobei die Impedanzbestimmungseinheit (70) konfiguriert ist zum Messen von die Impedanz der linken Herzkammer repräsentierenden Impedanzwerten über die Herzkammern bei gleichzeitigem Messen von die Impedanz der rechten Herzkammer repräsentierenden Impedanzwerten als Stellvertreter für das Herzschlagvolumen und wobei die Impedanzauswertungseinheit (78) konfiguriert ist zum Entfernen der die Impedanz der rechten Herzkammer repräsentierenden Impedanzwerte von einem Impedanzvektor oder einer Kombination von Impedanzvektoren, die Impedanzwerte umfasst, die die Impedanz sowohl der linken als auch der rechten Herzkammer repräsentieren, um die Impedanzänderungen auszuwählen, die auf dem Herzschlagvolumen der linken Herzkammer beruhen.

5. Herzstimulator nach Anspruch 1, wobei die Impedanzbestimmungseinheit (70) zusammen mit der Impedanzauswertungseinheit (78) konfiguriert ist zum automatischen Bestimmen der zur Bestimmung der Impedanzwerte verwendeten Elektroden.

6. Herzstimulator nach Anspruch 1, wobei die Impedanzauswertungseinheit (78) konfiguriert ist zum Berechnen der genannten mathematischen Kombination als eine gewichtete Summe.

7. Herzstimulator nach Anspruch 1, wobei die Impedanzauswertungseinheit (78) konfiguriert ist zum Berechnen der genannten mathematischen Kombination als Vektorkombination von Impedanzvektoren oder zum Berechnen der genannten mathematischen Kombination als Produkt von Impedanzvektoren.

8. Herzstimulator nach Anspruch 7, wobei die Impedanzauswertungseinheit (78) konfiguriert ist zum Berechnen eines Bereichs innerhalb der Vektorkombination von Impedanzvektoren durch Multiplizieren des Impedanzvektors zwischen einem ersten Elektrodenpaar mit dem Impedanzvektor zwischen einem zweiten Elektrodenpaar.

9. Herzstimulator nach Anspruch 8, wobei die Impedanzauswertungseinheit (78) konfiguriert ist zum Bestimmen von Gewichtungsfaktoren zum Bestimmen einer gewichteten Summe anhand des Bereichs innerhalb der Vektorenkombination von Impedanzvektoren oder zum Bestimmen eines zeitlichen Verlaufs des Bereichs innerhalb der Vektorkombination von Impedanzvektoren und zum Verwenden des Minimums und des Maximums des zeitlichen Verlaufs des Bereichs zum Bestimmen des relativen Herzschlagvolumens.

10. Herzstimulator nach Anspruch 1, wobei die Impedanzauswertungseinheit (78) konfiguriert ist zum Bestimmen eines Maximums der Änderung der mathematischen Kombination der Impedanzwerte im Verhältnis zur Zeit.

11. Herzstimulator nach einem der Ansprüche 7 bis 9, wobei die Impedanzauswertungseinheit (78) konfiguriert ist zum Berechnen des Bereichs unter einer die Änderung der Impedanz im Verhältnis zur Zeit beschreibenden Kurve und zum Auswerten des zeitlichen Verlaufs des Bereichs im Verhältnis zur Zeit.

12. Herzstimulator nach Anspruch 1, wobei die Impedanzauswertungseinheit (78) konfiguriert ist zum Auswerten der Zeitrelation der einzelnen Impedanzvektoren.

## Revendications

1. Stimulateur cardiaque (10) ayant une entrée électrique qui est reliée à une pluralité d'électrodes (18, 20, 22, 24, 31, 32, 33, 34, 36, 38, 40), ledit stimulateur cardiaque comprenant une unité de commande de stimulation (62), une ou plusieurs unités de stimulation (50, 52, 54), une unité de détermination d'impédance (70) et une unité d'évaluation d'impédance (78),
ladite une ou lesdites plusieurs unités de stimulation (50, 52, 54) étant configurée(s) pour générer des impulsions de stimulation et pour délivrer une impulsion de stimulation lorsqu'elles sont déclenchées par ladite unité de commande de stimulation (62),
ladite unité de commande de stimulation (62) étant de manière opérationnelle reliée à ladite une ou aux dites plusieurs unités de stimulation (50, 52, 54) pour commander des paramètres tels qu'un site de stimulation et/ou la synchronisation des impulsions de stimulation à délivrer par ladite une ou lesdites plusieurs unités de stimulation (50, 52, 54),
ladite unité de détermination d'impédance (70) étant configurée pour déterminer les valeurs d'impédance représentant l'impédance intracardiaque, et
ladite unité d'évaluation d'impédance (78) étant configurée pour évaluer lesdites valeurs d'impédance et délivrer un signal d'impédance évalué à ladite unité de commande de stimulation (62),
où ladite unité de détermination d'impédance (70) est configurée pour déterminer une pluralité de valeurs d'impédance entre différentes paires d'électrodes (18, 20, 22, 24, 31, 32, 33, 34, 36, 38, 40) reliées à ladite entrée électrique et où ladite unité d'évaluation d'impédance (78) est configurée pour générer une combinaison mathématique desdites valeurs d'impédance entre les différentes paires d'électrodes reliées à ladite entrée électrique et où ladite unité de commande de stimulation (62) est configurée pour déterminer les sites de stimulation respectifs et/ou la synchronisation des impulsions de stimulation en fonction de ladite combinaison mathématique,
**caractérisé en ce que**
l'unité d'évaluation d'impédance (78) est configurée pour générer ladite combinaison mathématique desdites valeurs d'impédance en tant que vecteurs d'impédance de manière géométrique, où l'unité de détermination d'impédance (70) est configurée pour déterminer les valeurs d'impédance par l'intermédiaire des paires d'électrodes de façon à ce que la surface concernée par les vecteurs d'impédance respectifs soit maximisée.

2. Stimulateur cardiaque selon la revendication 1, dans lequel ladite unité de commande de stimulation (62) est configurée pour déterminer un signal de volume qui représente une modification dans le volume du coeur entre la diastole et la systole, où le signal de volume est dérivé d'une amplitude de la combinaison mathématique desdites valeurs d'impédance au cours d'un cycle cardiaque respectif.

3. Stimulateur cardiaque selon la revendication 2, dans lequel ladite unité de commande de stimulation (62) est configurée pour déterminer les sites de stimulation respectifs et/ou la synchronisation des impulsions de stimulation en fonction dudit signal de volume de façon à ce que les sites de stimulation et la synchronisation des impulsions de stimulation conduisent à une modification maximale du volume entre la diastole et la systole et par conséquent à une maximisation de la sortie cardiaque.

4. Stimulateur cardiaque selon la revendication 1, dans lequel l'unité de détermination d'impédance (70) est configurée pour mesurer les valeurs d'impédance représentant l'impédance ventriculaire gauche à travers les ventricules tout en mesurant simultanément les valeurs d'impédance représentant l'impédance ventriculaire droite comme représentatives du volume systolique, et où l'unité d'évaluation d'impédance (78) est configurée pour supprimer les valeurs d'impédance représentant l'impédance ventriculaire droite du vecteur d'impédance ou d'une combinaison de vecteurs d'impédance qui englobe les valeurs d'impédance représentant à la fois l'impédance ventriculaire gauche et droite afin de sélectionner les modifications d'impédance qui sont dues au volume systolique ventriculaire gauche.

5. Stimulateur cardiaque selon la revendication 1, dans lequel l'unité de détermination d'impédance (70), conjointement avec l'unité d'évaluation d'impédance (78), est configurée pour déterminer les électrodes utilisées pour la détermination de manière automatique des valeurs d'impédance.

6. Stimulateur cardiaque selon la revendication 1, dans lequel l'unité d'évaluation d'impédance (78) est configurée pour calculer ladite combinaison mathématique sous la forme d'une somme pondérée.

7. Stimulateur cardiaque selon la revendication 1, dans lequel l'unité d'évaluation d'impédance (78) est configurée pour calculer ladite combinaison mathématique sous la forme d'une combinaison vectorielle des vecteurs d'impédance ou pour calculer ladite combinaison mathématique sous la forme d'un produit de vecteurs d'impédance.

8. Stimulateur cardiaque selon la revendication 7, dans lequel l'unité d'évaluation d'impédance (78) est configurée pour calculer une surface à l'intérieur de la combinaison vectorielle des vecteurs d'impédance en multipliant le vecteur d'impédance entre une première paire d'électrodes par le vecteur d'impédance entre une seconde paire d'électrodes.

9. Stimulateur cardiaque selon la revendication 8, dans lequel l'unité d'évaluation d'impédance (78) est configurée pour déterminer les facteurs de pondération afin de déterminer une somme pondérée par la surface à l'intérieur de la combinaison vectorielle des vecteurs d'impédance ou pour déterminer une évolution de la surface à l'intérieur de la combinaison vectorielle des vecteurs d'impédance et pour utiliser le minimum et le maximum de l'évolution de la surface pour déterminer le volume systolique relatif.

10. Stimulateur cardiaque selon la revendication 1, dans lequel l'unité d'évaluation d'impédance (78) est configurée pour déterminer un maximum de la modification de la combinaison mathématique des valeurs de l'impédance au fil du temps.

11. Stimulateur cardiaque selon la revendication 7 ou 9, dans lequel l'unité d'évaluation d'impédance (78) est configurée pour calculer la surface sous une courbe décrivant la modification de l'impédance au fil du temps et pour évaluer l'évolution de la surface au fil du temps.

12. Stimulateur cardiaque selon la revendication 1, dans lequel l'unité d'évaluation d'impédance (78) est configurée pour évaluer la relation temporelle des vecteurs d'impédance individuels.
